# DEMANDE DE BREVET EUROPEEN

(11) **EP 1 787 630 A2**
(43) Date de publication de la demande: **23.05.2007**
(21) Numéro de dépôt: 06121824.4
(22) Date de dépôt: 05.10.2006
(51) Int. Cl.: A61K 8/19, A61K 8/40

(54) **Composition cosmétique comprenant un pigment et/ou une charge préalablement traités en surface par un agent organique et un monomère électrophile**

(30) Priorité: 07.10.2005 FR 0553061
(71) Demandeur: L'Oreal, 75008 Paris (FR)
(72) Inventeur: BRUN, Gaëlle, 75015, PARIS (FR)
(74) Mandataire: Prevel, Estelle Nicole

(57) **Abrégé**

La présente invention a pour objet une composition pour le traitement des matières kératiniques, et en particulier des fibres kératiniques, comprenant, dans un milieu cosmétiquement acceptable, au moins un monomère électrophile et au moins un pigment et / ou une charge préalablement traités en surface par au moins un agent organique, ledit pigment ou ladite charge traité en surface étant différent d'un mica recouvert de titane et d'un pigment organique, un procédé de traitement des fibres kératiniques mettant en oeuvre une telle composition, ainsi que l'utilisation pour le traitement des fibres kératiniques d'une telle composition.

La composition conforme à la présente invention permet d'obtenir une coloration visible sur des fibres kératiniques foncées sans les dégrader et sans altérer leurs propriétés cosmétiques. De plus, la coloration obtenue présente une bonne résistance aux shampooings.

La composition conforme à l'invention permet d'obtenir un bon niveau de douceur sur les matières kératiniques, rémanent dans le temps et face aux shampooings, et homogène.

La composition conforme à l'invention présente une bonne stabilité.

## Description

La présente invention a pour objet une composition pour le traitement des matières kératiniques telles que la peau, les cheveux, les cils, les ongles, et en particulier des fibres kératiniques telles que les cheveux, comprenant, dans un milieu cosmétiquement acceptable, au moins un monomère électrophile et au moins un pigment et / ou une charge préalablement traités en surface par au moins un agent organique, ledit pigment ou ladite charge étant différent d'un mica recouvert de titane et d'un pigment organique.

Dans le domaine de la coloration des fibres kératiniques, il est déjà connu de colorer des fibres kératiniques par différentes techniques à partir de colorants directs ou de pigments pour des colorations non permanente ou de précurseurs de colorant pour des colorations permanentes.

La coloration non permanente ou coloration directe consiste à teindre les fibres kératiniques avec des compositions tinctoriales contenant des colorants directs. Ces colorants sont des molécules colorées et colorantes ayant une affinité pour les fibres kératiniques. Ils sont appliqués sur les fibres kératiniques pendant un temps nécessaire à l'obtention de la coloration désirée, puis rincés.

Les colorants classiques qui sont utilisés sont en particulier des colorants du type nitré benzénique, anthraquinonique, nitropyridinique, azoïque, xanthénique, acridinique, azinique, triarylméthane ou des colorants naturels.

Certains de ces colorants peuvent être utilisés dans des conditions éclaircissantes ce qui permet d'obtenir des colorations visibles sur des cheveux foncés.

Il est aussi connu de teindre les fibres kératiniques de façon permanente par la coloration d'oxydation. Cette technique de coloration consiste à appliquer sur les fibres kératiniques une composition contenant des précurseurs de colorant tels que des bases d'oxydation et des coupleurs. Ces précurseurs sous l'action d'un agent oxydant vont former dans le cheveu une ou plusieurs espèces colorées.

La variété des molécules mises en jeu au niveau des bases d'oxydation et des coupleurs, permet l'obtention d'une riche palette de couleurs et les colorations qui en résultent sont permanentes, puissantes, et résistantes aux agents extérieurs, notamment à la lumière, aux intempéries, aux lavages, à la transpiration et aux frottements.

Pour être visibles sur cheveux foncés, ces deux techniques de coloration nécessitent une décoloration préalable ou simultanée des fibres kératiniques. Cette étape de décoloration mise en oeuvre avec un agent oxydant tel que le peroxyde d'hydrogène ou les persels entraîne une dégradation non négligeable des fibres kératiniques ce qui altère leurs propriétés cosmétiques. Les cheveux ont alors tendance à devenir rêches, plus difficilement démêlables et plus fragiles.

Une autre méthode de coloration consiste à utiliser des pigments. En effet, l'utilisation de pigment à la surface des fibres kératiniques permet en général d'obtenir des colorations visibles sur cheveux foncés puisque le pigment en surface masque la couleur naturelle de la fibre. L'utilisation de pigment pour colorer des fibres kératiniques est par exemple décrite dans la demande de brevet FR 2 741 530, qui préconise l'utilisation pour la coloration des fibres kératiniques d'une composition comprenant au moins une dispersion de particules de polymère filmogène comportant au moins une fonction acide et au moins un pigment dispersé dans la phase continue de ladite dispersion.

Les colorations obtenues par ce mode de coloration présentent l'inconvénient d'avoir une faible résistance aux shampooings.

Dans le domaine du soin des matières kératiniques, il est connu d'utiliser des charges qui confèrent auxdites matières kératiniques des propriétés de douceur, de brillance et, dans le cas des cheveux, de volume et de lissage. Cependant, avec les méthodes de traitement classiques, les effets obtenus sont souvent insuffisants en terme d'efficacité et de durabilité.

Il est connu de la demande de brevet FR 2 833 489 des compositions de traitement des cheveux à partir de compositions comprenant des monomères électrophiles. Une telle composition permet d'obtenir des cheveux parfaitement gainés et non gras.

Il est également connu de l'art antérieur que la polymérisation des monomères électrophiles peut être accélérée par des pigments ou des charges. On peut citer à titre d'exemple le brevet US 5 290 825 qui décrit ce phénomène avec les alkyl cyanoacrylates.

Le but de la présente invention est de fournir des compositions pour le traitement des matières kératiniques, et en particulier des fibres kératiniques telles que les cheveux, qui permettent soit d'obtenir des colorations visibles sur support foncé sans qu'il soit nécessaire d'éclaircir ou de décolorer les fibres et qui présentent une bonne résistance aux shampooings, soit d'obtenir un effet de soin sur les matières kératiniques et en particulier les cheveux qui soit suffisamment marqué et durable dans le temps et face aux traitements classiques tels que les shampooings.

Ce but est atteint avec la présente invention qui a pour objet une composition pour le traitement des matières kératiniques comprenant, dans un milieu cosmétiquement acceptable, au moins un monomère électrophile et au moins un pigment et / ou une charge préalablement traités en surface par au moins un agent organique, ledit pigment ou ladite charge traité en surface étant différent d'un mica recouvert de titane et d'un pigment organique.

La composition conforme à la présente invention permet dans le cas des pigments d'améliorer la visibilité de la coloration sur une matière kératinique foncée. En particulier, dans le cas des fibres kératiniques foncées, on obtient une coloration très visible sans qu'il soit nécessaire d'éclaircir ou de décolorer les fibres kératiniques et par conséquent sans dégradation physique des fibres kératiniques.

De plus, la coloration obtenue présente une bonne résistance aux diverses agressions que peuvent subir les cheveux telles que les shampooings, les frottements, la lumière, les intempéries, la sueur et les déformations permanentes. Ces propriétés sont particulièrement remarquables en ce qui concerne la résistance de la coloration vis-à-vis des shampooings. La coloration est obtenue dans des nuances variées, elle est chromatique, puissante, esthétique et peu sélective. Les cheveux présentent par ailleurs de bonnes propriétés cosmétiques telles que la douceur ou un toucher naturel.

Dans le cas des charges, les compositions selon l'invention permettent d'obtenir un bon niveau de douceur sur les matières kératiniques, rémanent dans le temps et face aux shampooings. De plus, on constate une bonne homogénéité de l'effet sur le support kératinique.

Par ailleurs, le traitement en surface des pigments et / ou des charges présents dans la composition conforme à la présente invention permet d'obtenir une bonne stabilité de la composition conforme à l'invention.

La présente invention a également pour objet un procédé de traitement des fibres kératiniques mettant en oeuvre la composition conforme à l'invention, ainsi que l'utilisation de cette composition pour le traitement des fibres kératiniques.

La présente invention a aussi pour objet un kit de traitement comprenant d'une part un pigment et / ou une charge prélablement traités en surface par au moins un agent organique et d'autre part un monomère électrophile.

Dans le cadre de l'invention, le pigment ou la charge traité en surface est différent d'un mica recouvert de titane et d'un pigment organique, ce qui signifie que le pigment ou la charge traité en surface est différent d'un mica recouvert de titane traité en surface par un pigment organique.

Dans le cadre de l'invention, les pigments non traités en surface présentent une solubilité dans l'eau inférieure à 0,01 % à 20 °C, de préférence inférieure à 0,0001 %, et une absorption entre 350 et 700 nm, de préférence une absorption avec un maximum.

Le pigment non traité en surface, appelé par la suite pigment, peut-être un pigment organique. Par pigment organique, on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

Le ou les pigments organiques peuvent être choisis par exemple parmi le carmin, le noir de carbone, le noir d'aniline, la mélanine, le jaune azo, la quinacridone, le bleu de phtalocyanine, le rouge sorgho, les pigments bleus codifiés dans le Color Index sous les références Cl 42090, 69800, 69825, 73000, 74100, 74160, les pigments jaunes codifiés dans le Color Index sous les références Cl 11680, 11710, 15985, 19140, 20040, 21100, 21108, 47000, 47005, les pigments verts codifiés dans le Color Index sous les références Cl 61565, 61570, 74260, les pigments oranges codifiés dans le Color Index sous les réfénces Cl 11725, 15510, 45370, 71105, les pigments rouges codifiés dans le Color Index sous les références Cl 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

Ces pigments peuvent aussi être sous forme de pigments composites tels qu'ils sont décrits dans le brevet EP 1 184 426. Ces pigments composites peuvent être composés notamment de particules comportant un noyau inorganique recouvert au moins partiellement d'un pigment organique et au moins un liant assurant la fixation des pigments organiques sur le noyau.

A titre d'exemple, on peut aussi citer les pâtes pigmentaires de pigment organique telles que les produits vendus par la société HOECHST sous le nom :
- JAUNE COSMENYL IOG : Pigment YELLOW 3 (Cl 11710) ;
- JAUNE COSMENYL G : Pigment YELLOW 1 (Cl 11680) ;
- ORANGE COSMENYL GR : Pigment ORANGE 43 (Cl 71105) ;
- ROUGE COSMENYL R" : Pigment RED 4 (Cl 12085) ;
- CARMIN COSMENYL FB : Pigment RED 5 (Cl 12490) ;
- VIOLET COSMENYL RL : Pigment VIOLET 23 (Cl 51319) ;
- BLEU COSMENYL A2R : Pigment BLUE 15.1 (Cl 74160) ;
- VERT COSMENYL GG : Pigment GREEN 7 (Cl 74260) ;
- NOIR COSMENYL R Pigment BLACK 7 (Cl 77266).

Le pigment peut aussi être une laque. Par laque, on entend les colorants insolubilisés adsorbés sur des particules insolubles, l'ensemble ainsi obtenu restant insoluble lors de l'utilisation.

Les substrats inorganiques sur lesquels sont adsorbés les colorants sont par exemple l'alumine, la silice, le borosilicate de calcium et de sodium ou le borosilicate de calcium et d'aluminium, et l'aluminium.

Parmi les colorants organiques, on peut citer le carmin de cochenille. On peut également citer les produits connus sous les dénominations suivantes : D & C Red 21 (Cl 45 380), D & C Orange 5 (Cl 45 370), D & C Red 27 (Cl 45 410), D & C Orange 10 (Cl 45 425), D & C Red 3 (Cl 45 430), D & C Red 4 (Cl 15 510), D & C Red 33 (Cl 17 200), D & C Yellow 5 (Cl 19 140), D & C Yellow 6 (Cl 15 985), D & C Green (Cl 61 570), D & C Yellow 1 O (Cl 77 002), D & C Green 3 (Cl 42 053), D & C Blue 1 (Cl 42 090).

A titre d'exemples de laques, on peut citer le produit connu sous la dénomination suivante : D & C Red 7 (CI 15 850:1).

Le pigment peut aussi être un pigment à effets spéciaux. Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation, ...). Ils s'opposent par-là même aux pigments blancs ou colorés qui procurent une teinte uniforme opaque, semi-transparente ou transparente classique.

Il existe deux types de pigments à effets spéciaux, ceux à faible indice de réfraction tels que les pigments fluorescents, photochromes ou thermochromes, et ceux à plus fort indice de réfraction tels que les nacres ou les paillettes.

A titre de pigments à effets spéciaux, on peut citer les pigments nacrés tels que les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

Les quantum dots sont des nanoparticules semi conductrices luminescentes capables d'émettre, sous excitation lumineuse, un rayonnement présentant une longueur d'onde comprise entre 400 nm et 700 nm. Ces nanoparticules sont connues de la littérature. En particulier, elles peuvent être fabriqués selon les procédés décrits par exemple dans le US 6 225 198 ou US 5 990 479, dans les publications qui y sont citées, ainsi que dans les publications suivantes : Dabboussi B.O. et al "(CdSe)ZnS core-shell quantum dots : synthesis and characterisation of a size series of highly luminescent nanocristallites" Journal of phisical chemistry B, vol 101, 1997, pp 9463-9475*.* et Peng, Xiaogang et al, "Epitaxial Growth of highly Luminescent CdSe/CdS core/shell nanocrystals with photostability and electronic accessibility" Journal of the American Chemical Society, vol 119, N°30, pp 7019-7029*.*

Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques.

Le pigment peut être un pigment minéral. Par pigment minéral, on entend tout pigment qui répond à la définition de l'encyclopédie Ullmann dans le chapitre pigment inorganique. On peut citer, parmi les pigments minéraux utiles dans la présente invention, les oxydes de zirconium ou de cérium, ainsi que les oxydes de fer ou de chrome, le violet de manganèse, le bleu outremer, l'hydrate de chrome et le bleu ferrique, le dioxyde de titane. Les pigments minéraux suivants peuvent aussi être utilisés : Ta₂O₅, Ti₃O₅, Ti₂O₃, TiO, ZrO₂ en mélange avec TiO₂, ZrO₂, Nb₂O₅, CeO₂, ZnS.

Le pigment peut aussi être un pigment nacré tels que des pigments nacrés blancs par exemple le mica recouvert de titane, ou d'oxychlorure de bismuth, des pigments nacrés colorés tels que le mica recouvert de titane et d'oxydes de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth. A titre d'exemple, on peut citer les pigments Cellini commercialisée par Engelhard (Mica-TiO₂-laque), Prestige commercialisée par Eckart (Mica-TiO₂), Colorona commercialisée par Merck (Mica-TiO₂-Fe₂O₃).

En plus des nacres sur un support mica, on peut envisager les pigments multicouches basés sur des substrats synthétiques comme l'alumine, la silice, le borosilicate de calcium et de sodium ou le borosilicate de calcium et d'aluminium, et l'aluminium.

La taille du pigment utile dans le cadre de la présente invention est généralement comprise entre 10 nm et 200 µm, de préférence entre 20 nm et 80 µm, et plus préférentiellement entre 30 nm et 50 µm.

Par charge, on entend un composé substantiellement non coloré solide à température ambiante et pression atmosphérique, et insoluble dans les différents ingrédients de la composition, même lorsque ces ingrédients sont portés à une température supérieure à la température ambiante.

Les charges peuvent être minérales ou organiques. Les charges peuvent être des particules de toute forme, notamment plaquettaires, sphériques ou oblongues, quelle que soit leur forme cristallographique (par exemple feuillet, cubique, hexagonale, orthorhombique).

Parmi les charges utilisables dans les compositions selon l'invention, on peut notamment citer le talc ; le mica naturel ou synthétique ; la silice ; le kaolin ; les poudres de polyamide (Nylon^{®}), de poly-β-alanine et de polyéthylène ; les poudres de polymères de tétrafluoroéthylène (Téflon^{®}) ; la lauroyl-lysine ; l'amidon ; le nitrure de bore ; les poudres de polymères d'acide acrylique ; les microbilles de résine de silicone comme par exemple les « TOSPEARLS^{®} » de la société TOSHIBA ; les oxychlorures de bismuth ; le carbonate de calcium précipité ; le carbonate et l'hydro-carbonate de magnésium ; l'hydroxyapatite ; les microsphères de silice creuses telles que les 'SILICA BEADS SB 700/HA^{®}' ou 'SILICA BEADS SB 700^{®}' de la société MAPRECOS, les 'SUNSPHERES H-33^{®}' et les 'SUNSPHERES H-51^{®}' de la société ASAHI GLASS ; les microsphères de polymères acryliques telles que celles en copolymère d'acrylate réticulé 'POLYTRAP 6603 ADSORBER^{®}' de la société RP SCHERRER et celles de polyméthacrylate de méthyle 'MICROPEARL M 100^{®}' de la société SEPPIC ; les poudres de polyuréthane telle que la poudre de copolymère de diisocyanate d'hexaméthylène et de triméthylol hexyl lactone vendue sous la dénomination « PLASTIC POWDER D-400^{®}» par la société TOSHIKI ; les microcapsules de verre ou de céramique ; les savons métalliques dérivés d'acides organiques carboxyliques ayant de 8 à 22 atomes de carbone, notamment de 12 à 18 atomes de carbone, par exemple le stéarate de zinc, de magnésium ou de lithium, le laurate de zinc, le myristate de magnésium ; les microcapsules de polymères ou de copolymères d'acrylate ou de méthacrylate de méthyle, ou encore de copolymères de chlorure de vinylidène et d'acrylonitrile, comme l' « EXPANCEL^{®}» de la société EXPANCEL; les poudres d'organopolysiloxane réticulés élastomères telles que celles vendues sous la dénomination « TREFIL POWDER E-506C » par la société DOW CORNING ; et leurs mélanges.

Les charges traitées en surface ou non peuvent avoir un diamètre apparent allant de 0,01 à 150 µm et mieux de 0,5 à 150 µm. Un diamètre apparent correspond au diamètre du cercle dans lequel s'inscrit la particule élémentaire selon sa plus petite dimension (épaisseur pour des lamelles).

Les pigments et les charges préalablement traités en surface utiles dans le cadre de l'invention sont des pigments ou des charges qui ont subi totalement ou partiellement un traitement de surface de nature chimique, électronique, électro-chimique, mécano-chimique ou mécanique, avec un agent organique tel que ceux qui sont décrits notamment dans Cosmetics and Toiletries, Février 1990, Vol. 105, p. 53-64 avant d'être dispersés dans la composition conforme à l'invention. Ces agents organiques peuvent être par exemple choisis parmi les acides aminés ; les cires, par exemple la cire de carnauba et la cire d'abeille ; les acides gras, les alcools gras et leurs dérivés, tels que l'acide stéarique, l'acide hydroxystéarique, l'alcool stéarylique, l'alcool hydroxystéarylique, l'acide laurique et leurs dérivés ; les tensio-actifs anioniques ; les lécithines ; les sels de sodium, potassium, magnésium, fer, titane, zinc ou aluminium d'acides gras, par exemple le stéarate ou laurate d'aluminium ; les alcoxydes métalliques ; les polysaccharides, par exemple le chitosane, la cellulose et ses dérivés ; le polyéthylène ; les polymères (méth)acryliques, par exemple les polyméthylmethacrylates ; les polymères et copolymères contenant des motifs acrylates ; les protéines ; les alcanoamines ; les composés siliconés, par exemple les silicones, les polydiméthylsiloxanes, les alcoxysilanes, les alkylsilanes, les siloxy-silicates ; les composés organiques fluorés, par exemple les perfluoroalkyle éthers ; les composés fluoro-siliconés.

Le traitement en surface au sens de la présente invention est tel qu'un pigment traité en surface conserve ses propriétés intrinsèques de pigment avant traitement et une charge traitée en surface conserve ses propriétés intrinsèques de charge avant traitement. Par exemple, les substrats inorganiques tels que l'alumine ou la silice sur lesquels sont adsorbés des colorants organiques ne sont pas des charges traitées en surface au sens de la présente invention.

Les pigments et les charges traités en surface utiles dans le cadre de l'invention peuvent aussi avoir été traités par un mélange de ces composés et / ou avoir subi plusieurs traitements de surface.

Les pigments et les charges traités en surface utiles dans le cadre de la présente invention peuvent être préparés selon des techniques de traitement de surface bien connues de l'homme de l'art ou trouvés tels quels dans le commerce.

De préférence, les pigments et / ou les charges traités en surface sont recouverts par une couche organique.

L'agent organique avec lequel sont traités les pigments et les charges peut être déposé sur les pigments ou les charges par évaporation de solvant, réaction chimique entre les molécules de l'agent de surface ou création d'une liaison covalente entre l'agent de surface et les pigments ou les charges.

Le traitement en surface peut ainsi être réalisé par exemple par réaction chimique d'un agent de surface avec la surface des pigments ou des charges et création d'une liaison covalente entre l'agent de surface et les pigments ou les charges. Cette méthode est notamment décrite dans le brevet US 4 578 266.

De préférence, on utilisera un agent organique lié aux pigments ou aux charges de manière covalente.

L'agent pour le traitement de surface peut représenter de 0,1 à 50 % en poids du poids total des pigments ou des charges traités en surface, de préférence de 0,5 à 30 % en poids, et encore plus préférentiellement de 1 à 10 % en poids.

De préférence, les traitements en surface des pigments et des charges sont choisis parmi les traitements suivants :
- un traitement PEG-Silicone comme le traitement de surface AQ commercialisé par LCW ;
- un traitement Chitosane comme le traitement de surface CTS commercialisé par LCW ;
- un traitement Triéthoxycaprylylsilane comme le traitement de surface AS commercialisé par LCW ;
- un traitement Méthicone comme le traitement de surface SI commercialisé par LCW ;
- un traitement Diméthicone comme le traitement de surface Covasil 3.05 commercialisé par LCW ;
- un traitement Diméthicone / Triméthylsiloxysilicate comme le traitement de surface Covasil 4.05 commercialisé par LCW ;
- un traitement Lauroyl Lysine comme le traitement de surface LL commercialisé par LCW ;
- un traitement Lauroyl Lysine Diméthicone comme le traitement de surface LL / SI commercialisé par LCW ;
- un traitement Myristate de Magnésium comme le traitement de surface MM commercialisé par LCW ;
- un traitement Dimyristate d'Aluminium comme le traitement de surface MI commercialisé par Miyoshi ;
- un traitement Perfluoropolyméthylisopropyl éther comme le traitement de surface FHC commercialisé par LCW ;
- un traitement Isostéaryl Sébacate comme le traitement de surface HS commercialisé par Miyoshi ;
- un traitement Disodium Stéaroyl Glutamate comme le traitement de surface NAI commercialisé par Miyoshi ;
- un traitement Diméthicone / Disodium Stéaroyl Glutamate comme le traitement de surface SA / NAI commercialisé par Miyoshi ;
- un traitement Phosphate de Perfluoroalkyle comme le traitement de surface PF commercialisé par Daito ;
- un traitement Copolymère acrylate / Diméthicone et Phosphate de Perfluoalkyle comme le traitement de surface FSA commercialisé par Daito ;
- un traitement Polyméthylhydrogène siloxane / Phosphate de Perfluoroalkyle comme le traitement de surface FS01 commercialisé par Daito ;
- un traitement Lauryl Lysine / Aluminium Tristéarate comme le traitement de surface LL-StAl commercialisé par Daito ;
- un traitement Octyltriéthylsilane comme le traitement de surface OTS commercialisé par Daito ;
- un traitement Octyltriéthylsilane / Phosphate de Perfluoroalkyle comme le traitement de surface FOTS commercialisé par Daito ;
- un traitement Copolymère Acrylate / Diméthicone comme le traitement de surface ASC commercialisé par Daito ;
- un traitement Isopropyl Titanium Triisostéarate comme le traitement de surface ITT commercialisé par Daito ;
- un traitement Cellulose Microcrystalline et Carboxyméthyl Cellulose comme le traitement de surface AC commercialisé par Daito ;
- un traitement Cellulose comme le traitement de surface C2 commercialisé par Daito ;
- un traitement copolymère Acrylate comme le traitement de surface APD commercialisé par Daito ;
- un traitement Phosphate de Perfluoroalkyle / Isopropyl Titanium Triisostéarate comme le traitement de surface PF + ITT commercialisé par Daito.

Le ou les pigments traités en surface sont généralement présents dans la composition dans des quantités totales généralement comprises entre 0,05 et 50 % en poids du poids total de la composition, de préférence entre 0,1 et 35 % en poids, encore plus préférentiellement entre 0,5 et 20 % en poids.

La ou les charges traitées en surface ou non sont généralement présentes dans la composition dans des quantités totales comprises entre 0,05 et 95 % en poids du poids total de la composition, de préférence de 0,1 à 50 % en poids, encore plus préférentiellement de 0,5 à 30 % en poids.

La composition conforme à la présente invention peut de plus comprendre une ou plusieurs charges non traitées en surface.

La composition conforme à la présente invention peut de plus comprendre un ou plusieurs pigments non traités en surface.

Par monomère électrophile, on entend un monomère capable de polymériser par polymérisation anionique en présence d'un agent nucléophile.

Par polymérisation anionique, on entend le mécanisme défini dans l'ouvrage « Advanced Organic Chemistry », Third Edition de Jerry March, pages 151 à 161.

Les agents nucléophiles susceptibles d'initier la polymérisation anionique sont des systèmes connus capables de générer un carbanion au contact d'un agent électrophile, tels que les ions hydroxyde contenus dans l'eau à pH neutre. On entend par carbanion les espèces chimiques définies dans l'ouvrage « Advanced Organic Chemistry », Third Edition de Jerry March, page 141.

Le ou les monomères électrophiles présents dans la composition de l'invention peuvent par exemple être choisis parmi :
- les dérivés benzylidène malononitrile (A), le 2-(4-chloro-benzylidène)-malononitrile (A1), le 2-cyano-3-phényl acrylate d'éthyle (B), le 2-cyano-3-(4-chloro-phényl) acrylate d'éthyle (B1) décrits dans Sayyah, J. Polymer Research, 2000, p. 97 : 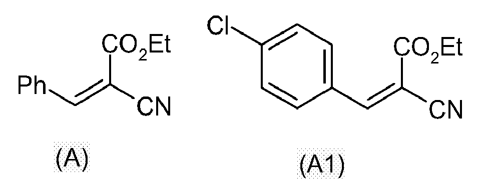 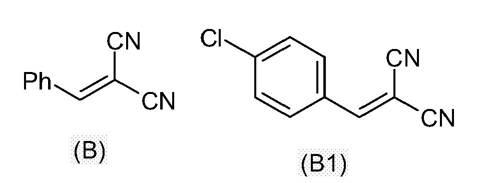
- les dérivés de méthylidènemalonate comme le 2-méthylène-malonate de diéthyle (C) décrits par Hopff, Makromoleculare Chemie, 1961, p. 95, De Keyser, J. Pharm. Sci, 1991, p. 67 et Klemarczyk, Polymer, 1998, p. 173 : 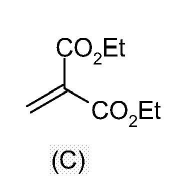
- le 2-éthoxycarbonylméthyleneoxycarbonyl acrylate d'éthyle (D) décrit par Breton, Biomaterials, 1998, p. 271 et Couvreur, Pharmaceutical Research, 1994, p. 1270 : 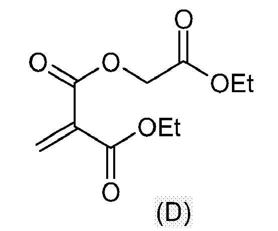
- les dérivés itaconate et itaconimide comme l'itaconate de diméthyle (E) par Bachrach, European Polymer Journal, 1976, p. 563, le N-butyl itaconimide (F), le N-(4-tolyl) itaconimide (G), le N-(2-éthylphényl) itaconimide (H), le N-(2,6-diéthylphényl) itaconimide (I) décrits par Wanatabe, J. Polymer Science : Part A : Polymer chemistry, 1994, p. 2073 : 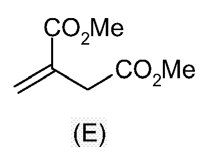 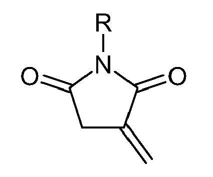 R= Butyle (F), 4-tolyle (G), 2-éthylphényle (H), 2,6-diéthyphényle (I)
- les dérivés α-(méthylsulfonyl)acrylates de méthyle (K), α-(méthylsulfonyl)acrylates de d'éthyle (L), α-(tert-butylsulfonyl)acrylates de méthyle (M), α-(méthylsulfonyl)acrylates de tert-butyle (N), α-(tert-butylsulfonyl)acrylates de tert-butyle (O) décrits par Gipstein, J.Org.Chem, 1980, p. 1486, et
- les dérivés 1,1-bis-(méthylsulfonyl)éthylène (P), 1-acétyl-1-méthyl sulfonyl éthylène (Q), α-(méthylsulfonyl) vinyl sulfonate de méthyle (R), α-méthylsulfonylacrylonitrile (S) décrits par Shearer dans le brevet US 2 748 050 : 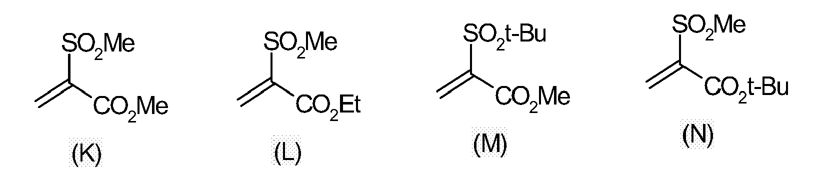 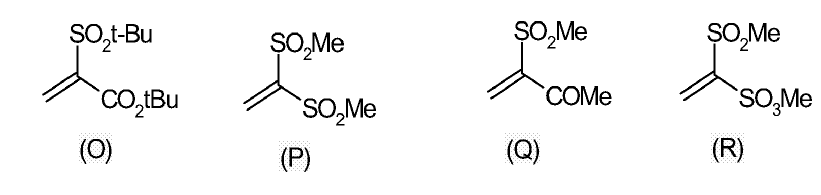 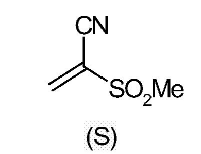
- les dérivés méthyl vinyl sulfone (T) et phényl vinyl sulfone (U) décrits par Boor, J.Polymer Science, 1971, p. 249 : 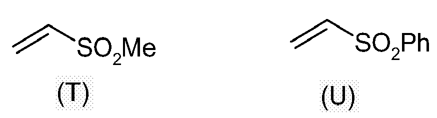
- le dérivé phényl vinyl sulfoxide (V) décrit par Kanga, Polymer preprints (ACS, Divison of Polymer Chemistry), 1987, p. 322 : 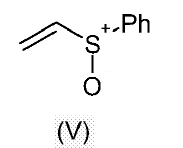
- le dérivé 3-méthyl-N-(phénylsulfonyl)-1-aza-1,3-butadiène (W) décrit par Bonner, Polymer Bulletin, 1992, p. 517 : 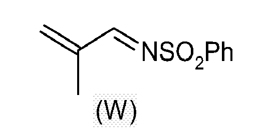
- les dérivés acrylates et acrylamides comme :
   - le N-propyl-N-(3-triisopropoxysilylpropyl)acrylamide (X) et le N-propyl-N-(3-triéthoxysilylpropyl)acrylamide (Y) décrits par Kobayashi, Journal of Polymer Science, Part A : Polymer Chemistry, 2005, p. 2754 : 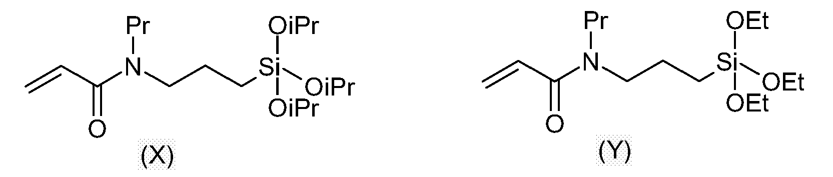
   - le 2-hydroxyéthyl acrylate (Z) et le 2-hydroxyéthyl méthacrylate (AA) décrits par Rozenberg, International Journal of Plastics Technology, 2003, p. 17 : 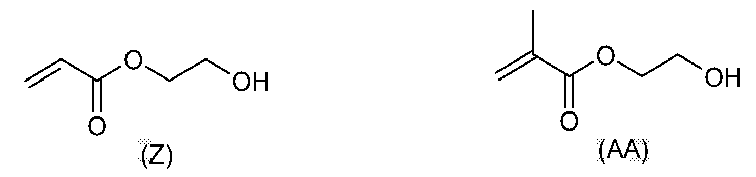
   - le N-butyl acrylate (AB) décrit par Schmitt, Macromolecules, 2001, p. 2115 et le tert-butyl acrylate (AC) décrit par lshizone, Macromolecules, 1999, p. 955 :

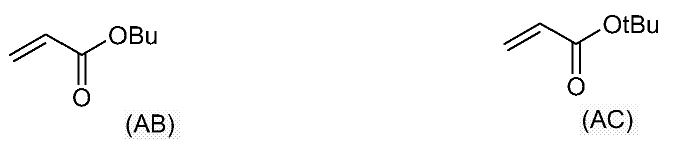

Le monomère électrophile utile dans la présente invention peut être cyclique ou linéaire. Lorsqu'il est cyclique, le groupe éléctro-attracteur est de préférence exocyclique, c'est-à-dire qu'il ne fait pas partie intégrante de la structure cyclique du monomère.

Selon un mode de réalisation particulier de l'invention, le monomère électrophile utile dans le cadre de l'invention présente au moins deux groupes électro-attracteurs.

Selon un mode de réalisation préféré de l'invention, le ou les monomères électrophiles sont choisis parmi les composés de formule (I) : 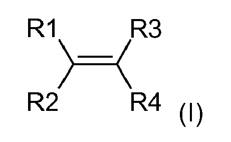 dans laquelle :
- R₁ et R₂ désignent chacun, indépendamment l'un de l'autre, un groupe peu ou non électro-attracteur (peu ou non inductif-attracteur) tel que :
   - un atome d'hydrogène,
   - un groupe hydrocarboné saturé ou non, linéaire, ramifié ou cyclique, aromatique ou non, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène,
   - un résidu polyorganosiloxane modifié ou non,
   - un groupement polyoxyalkylène ;
- R₃ et R₄ désignent chacun, indépendamment l'un de l'autre, un groupe électro-attracteur (ou inductif-attacteur) choisi de préférence parmi les groupements -N(R)₃⁺, -S(R)₂⁺, -SH₂⁺, -NH₃⁺, -NO₂, -SO₂R, -C≡N, -COOH,-COOR, -COSR, -CONH₂, -CONHR, -F, -Cl, -Br, -I, -OR, -COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle tels que phényle, ou les groupements aryloxy tels que phénoxyloxy ;
- R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR',-OH, les atomes d'halogène, ou un résidu de polymère pouvant être obtenu par polymérisation radicalaire, par condensation ou par ouverture de cycle, R' désignant un radical alkyle en C₁-C₁₀.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, mieux encore de 1 à 10 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que -(CH₂)ₙ-(CF₂)ₘ-CF₃ ou-(CH₂)ₙ-(CF₂)ₘ-CHF₂ avec n=1 à 20 et m= 1 à 20.

Les substituants R₁ à R₄ peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoiques, quinoniques, méthiniques, cyanométhiniques et triarylméthane.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et de type esters gras.

Selon un mode de réalisation préféré de l'invention, le ou les monomères électrophiles sont choisis parmi les monomères de la famille des cyanoacrylates de formule (II) : 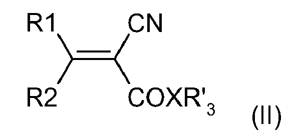 dans laquelle :
- X peut désigner NH, S, O ;
- R'₃ peut désigner un atome d'hydrogène ou un groupe R ;
- R, R₁ et R₂ sont tels que définis précédemment.

Dans les formules (I) et (II), R₁ et R₂ représentent de préférence un atome d'hydrogène.

Dans la formule (II), X désigne de préférence O et R'₃ représente de préférence un radical alkyle en C₆-C₁₀.

A titre de composés de formule (II), on peut citer les monomères :
a) appartenant à la famille des 2-cyanoacrylates de polyfluoroalkyle tels que l'ester 2,2,3,3-tétrafluoropropylique de l'acide 2-cyano-2-propénoïque de formule (III) : 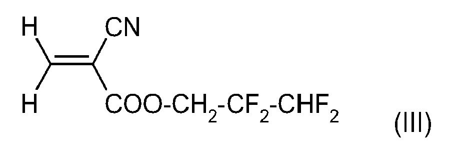 ou encore l'ester 2,2,2-trifluoroéthylique de l'acide 2-cyano-2-propénoïque de formule (IV) : 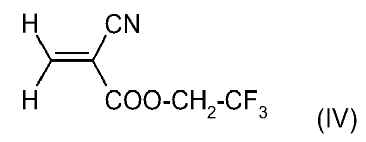
b) appartenant à la famille des 2-cyanoacrylates d'alkyle ou d'alcoxyalkyle de formule (VI) : 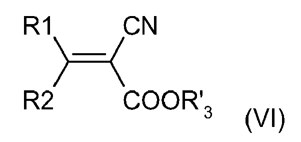 dans laquelle :
   - R'₃ représente un radical alkyle en C₁-C₁₀, alcényle en C₂-C₁₀ ou alcoxy(C₁-C₄)alkyle(C₁-C₁₀), de préférence un radical alkyle en C₁-C₁₀ ou alcoxy(C₁-C₄)alkyle(C₁-C₁₀) ;
   - R₁ et R₂ sont tels que définis précédemment.

On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle, le 2-cyanoacrylate d'allyle, le 2-cyanoacrylate de méthoxypropyle et le cyanoacrylate d'iso-amyle.

Dans la formule (VI) telle que définie ci-dessus, R'₃ représente de préférence un radical alkyle en C₆-C₁₀, et R₁ et R₂ représentent un atome d'hydrogène.

Dans le cadre de l'invention, on préfère utiliser les monomères définis en b).

Les monomères le plus particulièrement préféré sont les composés de formule (VII) et leurs mélanges : 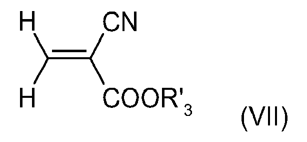 dans laquelle R'₃ est choisi parmi les radicaux suivants :
-(CH₂)₇-CH₃ ;
-CH(CH₃)-(CH₂)₅-CH₃ ;
-CH₂-CH(C₂H₅)-(CH₂)₃-CH₃ ;
-(CH₂)₅-CH(CH₃)-CH₃ ;
-(CH₂)₄-CH(C₂H₅)-CH₃.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Le ou les monomères électrophiles sont généralement présents dans la composition conforme à l'invention en quantité comprise entre 0,1 % et 80 % en poids environ du poids total de la composition, et de préférence entre 1 % et 50 % en poids environ du poids total de la composition.

Le ou les monomères électrophiles utiles dans le cadre de la présente invention peuvent être synthétisés selon les méthodes connues décrites dans la littérature. En particulier, les monomères de la famille des cyanoacrylates peuvent être synthétisés selon l'enseignement des documents US 3 527 224, US 3 591 767, US 3 667 472, US 3 995 641, US 4 035 334 et US 4 650 826.

Selon la présente invention, les monomères sont de préférence choisis parmi les monomères capables de polymériser sur les fibres kératiniques dans des conditions cosmétiquement acceptables. En particulier, la polymérisation du monomère s'effectue de préférence à une température inférieure ou égale à 80 °C, de préférence entre 10 et 80 °C, de préférence entre 20 et 80 °C, ce qui n'empêche pas de terminer l'application par un séchage au casque, un brushing ou passage au fer plat ou à friser.

Le ou les agents nucléophiles peuvent être appliqués indépendamment de la composition de l'invention. Ils peuvent aussi être ajoutés à la composition de l'invention au moment de l'emploi. Dans ce cas-là, la composition conforme à l'invention comprend de plus au moins un agent nucléophile.

Selon un mode de réalisation particulier de l'invention, le ou les agents nucléophiles sont choisis parmi las composés moléculaires, les oligomères, les dendrimères et les polymères portant au moins une fonction nucléophile choisie parmi les fonctions R₂N⁻, NH₂⁻, Ph₃C⁻, R₃C⁻, PHNH⁻, pyridine, ArS⁻, R-C≡C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻, H₂O, Ph représentant un groupe phényle, Ar représentant un groupe aryle et R représentant un groupe alkyle en C₁-C₁₀.

De préférence, l'agent nucléophile est l'eau.

Le milieu cosmétiquement acceptable de la composition de l'invention est de préférence sous forme d'un milieu anhydre et non hygroscopique. On entend par milieu anhydre un milieu contenant moins de 1 % d'eau.

Le milieu cosmétiquement acceptable de la composition de l'invention est de préférence choisi parmi :
- les alcools aromatiques tels que l'alcool benzylique ;
- les alcools gras ;
- les polyols modifiés ou non tels que le glycérol, le glycol, le propylène glycol, le dipropylène glycol, le butylène glycol, le butyle diglycol ;
- les silicones volatiles ou non telles que la cyclopentasiloxane, la cyclohexasiloxane, les polydiméthylsiloxanes modifiées ou non par des fonctions phényle et / ou siloxy et / ou silanol et / ou amine et / ou imine et / ou fluoroalkyle et / ou carboxylique et / ou bétaïne et / ou ammonium quaternaire ;
- les huiles minérales, organiques ou végétales ;
- les cires oxyéthylénées ou non, les paraffines, les alcanes et plus particulièrement les alcanes en C₅-C₁₀ ;
- les acides gras, les amides grasses, les esters gras et plus particulièrement les benzoates ou les salicylates d'alcool gras ; et leurs mélanges.

Le milieu cosmétiquement acceptable de la composition de l'invention peut aussi se présenter sous forme d'une émulsion directe ou inverse et / ou être encapsulé, les monomères électrophiles étant maintenus dans un milieu anhydre jusqu'au moment de l'emploi. La phase dispersée ou continue de l'émulsion peut être constituée par de l'eau, un alcool aliphatique en C₁-C₄, des silicones ou leurs mélanges. Les capsules ou microcapsules contenant la composition de l'invention peuvent être dispersées dans un milieu anhydre tel que défini précédemment, de l'eau, un alcool aliphatique en C₁-C₄ ou leurs mélanges.

La composition conforme à l'invention peut de plus comprendre au moins un inhibiteur de polymérisation.

De préférence, le ou les inhibiteurs de polymérisation sont des inhibiteurs de polymérisation anionique et / ou radicalaire.

A titre d'exemples d'inhibiteurs de polymérisation anionique et / ou radicalaire, on peut citer le dioxyde de soufre, l'oxyde nitrique, les acides organiques tels qu'un acide sulfonique, l'acide phosphorique ou l'acide acétique, la lactone, le trifluorure de bore, l'hydroquinone et ses dérivés tels que l'hydroquinone monoéthyléther, la tertiobutyl hydroquinone, la benzoquinone et ses dérivés tels que la duroquinone, le catéchol et ses dérivés tels que le t-butyl catéchol et le méthoxycatéchol, l'anisole et ses dérivés tels que le méthoxyanisole ou l'hydroxyanisole, le pyrogallol et ses dérivés, le p-méthoxyphénol, l'hydroxybutyl toluène, les alkyl sulfates, les alkyl sulfites, les alkyl sulfones, les alkyl sulfoxydes, les alkyl sulfures, les mercaptans, le 3-sulfonène et leurs mélanges. Les groupements alkyle désignent de préférence des groupements ayant de 1 à 6 atomes de carbone.

Le ou les inhibiteurs de polymérisation sont généralement présents dans la composition conforme à l'invention en quantité comprise entre 0,01 % et 10 % en poids environ du poids total de la composition, et encore plus préférentiellement entre 0,05 % et 5 % en poids environ du poids total de la composition.

La composition conforme à l'invention peut de plus comprendre au moins un polymère épaississant ne présentant pas de réactivité sur le ou les monomères électrophiles utiles dans le cadre de la présente invention.

A titre d'exemples de polymères épaississants ne présentant pas de réactivité sur le ou les monomères électrophiles utilisés dans le cadre de l'invention, on peut citer de façon non exhaustive le polyméthylméthacrylate (PMMA) ou encore les copolymères à base de cyanoacrylate tels qu'ils sont décrits dans le brevet US 6 224 622.

Le ou les polymères épaississants sont généralement présents dans la composition conforme à l'invention en quantité comprise entre 0,1 % et 50 %, de préférence entre 0,5 % et 25 %. Ils sont entre autres utiles pour moduler la vitesse de polymérisation des monomères électrophiles.

La composition conforme à l'invention peut également contenir des composés additionnels utilisés classiquement en cosmétique. Ces composés peuvent être choisis parmi les agents réducteurs, les corps gras, les silicones organomodifiées ou non, les agents épaississants autres que les polymères épaississants définis ci-dessus, les polymères cationiques, anioniques, neutres ou amphotères, les adoucissants, les agents anti-mousse, les agents hydratants, les agents émollients, les agents alcalinisants, les agents anti-oxydants, les agents anti-radicaux libres, les agents chélatants, les agents antipelliculaires, les agents régulateurs de séborrhée, les agents apaisants, les plastifiants, les filtres solaires, les colorants directs (naturels ou non) ou d'oxydation (bases et / ou coupleurs), les pigments, les charges minérales, les argiles, les minéraux colloïdaux, les nacres, les parfums, les peptisants, les conservateurs, les tensioactifs anioniques, cationiques, amphotères, zwittérioniques ou non-ioniques, les polymères fixants ou non, les polymères conditionneurs, les protéines hydrolysées ou non, les enzymes, les acides aminés, les oligopeptides, les peptides, les vitamines, les saccharides, les oligosaccharides, les polysaccharides hydrolysés ou non, modifiés ou non, les polyacides amines, les alcools gras ramifiés ou non, les cires animales, végétales ou minérales, les céramides, les pseudo-céramides, les acides organiques hydroxylés, les polyisobutènes et poly(α-oléfines), les esters gras, les polymères anioniques sous forme dissoute ou dispersée, les polymères non ioniques sous forme dissoute ou dispersée.

Bien entendu, l'homme de l'art veillera à choisir ce ou ces éventuels composés additionnels de manière telle que les propriétés avantageuses attachées intrinsèquement à la composition conforme à l'invention ne soient pas, ou substantiellement pas, altérer par la ou les adjonctions envisagées.

La composition conforme à l'invention peut se présenter sous des formes diverses, telles que des lotions, des sprays, des mousses et être appliquées sous forme de shampooing ou d'après-shampooing.

La composition conforme à l'invention peut également contenir un propulseur. Le propulseur est généralement constitué par un gaz comprimé ou liquéfié usuellement employé pour la préparation de compositions aérosols. De préférence, le gaz comprimé ou liquéfié est choisi parmi l'air, le gaz carbonique, l'azote comprimé, un gaz soluble tel que le diméthyléther, les hydrocarbures halogénés tels que les hydrocarbures fluorés ou non halogénés, et leurs mélanges.

La composition conforme à l'invention peut se présenter telle quelle ou résulter du mélange au moment de l'emploi de deux compositions. Par exemple, la première composition peut comprendre le ou les monomères électrophiles et la deuxième composition peut comprendre le ou les pigments et / ou la ou les charges traités en surface.

Le procédé selon la présente invention comprend l'application sur les fibres kératiniques d'une composition comprenant, dans un milieu cosmétiquement acceptable, au moins un pigment et / ou une charge préalablement traités en surface et au moins un monomère électrophile telle que définie précédemment en présence d'au moins un agent nucléophile.

Selon une variante du procédé de l'invention, l'application de la composition conforme à l'invention est réalisée en au moins deux étapes, une des étapes consiste à appliquer sur les fibres kératiniques une composition comprenant le ou les pigments et / ou la ou les charges traités en surface et l'autre étape consiste à appliquer sur les fibres kératiniques une composition comprenant le ou les monomères électrophiles, l'ordre des étapes étant indifférent.

L'agent nucléophile utile peut être utilisé pur, en solution, sous forme d'une émulsion ou être encapsulé. Il peut être présent dans la composition contenant le pigment traité en surface. Il peut aussi être appliqué séparément. Dans ce cas, il est possible par exemple d'imprégner préalablement les fibres kératiniques à l'aide de cet agent nucléophile.

Dans le cas où l'agent nucléophile est de l'eau, il est possible d'humidifier préalablement les fibres kératiniques à l'aide d'une solution aqueuse dont le pH a été ajusté à l'aide d'agents acidifiants et / ou alcalinisants.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VII) suivante : 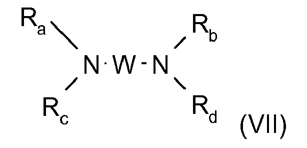 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

Selon un mode de réalisation particulier de l'invention, on augmente la nucléophilie des fibres kératiniques par transformation chimique de la matière kératinique. A titre d'exemple, on peut appliquer sur les fibres kératiniques, avant l'application de la composition conforme à l'invention telle que définie précédemment, au moins un agent réducteur de la kératine afin de réduire les ponts disulfure composant en partie la kératine en thiols.

Parmi les agents réducteurs de la kératine utiles, on peut citer :
- le thiosulfate de sodium anhydre ;
- le métabisulfite de sodium en poudre ;
- la thiourée ;
- le sulfite d'ammonium ;
- l'acide thioglycolique ;
- l'acide thiolactique ;
- le thiolactate d'ammonium ;
- le mono-thioglycolate de glycérol ;
- le thioglycolate d'ammonium ;
- le thioglycérol ;
- l'acide 2,5-dihydroxybenzoique ;
- le di-thioglycolate de diammonium ;
- le thioglycolate de strontium ;
- le thioglycolate de calcium ;
- le formo-sulfoxylate de zinc ;
- le thioglycolate d'isooctyle ;
- la dl-cystéine ;
- le thioglycolate de monoéthanolamine.

Afin d'améliorer entre autres l'adhésion du polycyanoacrylate formé in situ, on peut préalablement traiter les fibres kératiniques avec tous types de polymères, ou réaliser un traitement capillaire, comme une coloration directe ou d'oxydation, une permanente ou un défrisage.

L'application des compositions sur les fibres kératiniques peut être suivie ou non d'un rinçage et / ou d'un séchage. Le séchage peut être effectué au casque, au sèche cheveux et / ou au fer à lisser.

Ces compositions peuvent en outre contenir divers composés additionnels tels que définis précédemment.

Selon le procédé conforme à l'invention, il est possible de réaliser des superpositions multiples d'applications.

La présente invention a aussi pour objet un kit pour le traitement des fibres kératiniques, caractérisé par le fait qu'il contient une composition comprenant au moins un pigment et / ou une charge préalablement traités en surface tel que défini précédemment et une composition comprenant au moins un monomère électrophile tel que défini précédemment et éventuellement au moins un inhibiteur de polymérisation.

Selon un mode de réalisation particulier du kit conforme à l'invention, la composition comprenant le ou les pigments et / ou la ou les charges traités en surface comprend de plus au moins un agent nucléophile tel que défini précédemment.

Selon un autre mode de réalisation particulier du kit conforme à l'invention, le kit contient de plus une composition comprenant au moins un agent nucléophile tel que défini précédemment.

Selon une première variante de l'invention, la composition comprenant le ou les pigments et / ou le ou les charges traités en surface et la composition comprenant le ou les monomères électrophiles et éventuellement le ou les inhibiteurs de polymérisation sont présentes dans une seule composition anhydre.

Selon une deuxième variante, la composition comprenant le ou les pigments et / ou la ou les charges traités en surface est une composition aqueuse et la composition comprenant le ou les monomères électrophiles et éventuellement le ou les inhibiteurs de polymérisation est une composition anhydre.

Le ou les monomères électrophiles sont présents dans la composition les comprenant dans une concentration généralement comprise entre 0,05 et 30 % en poids environ du poids total de la composition, de préférence entre 0,01 et 50 % en poids, et encore plus préférentiellement entre 0,1 et 20 % en poids.

La présente invention a également pour objet l'utilisation pour le traitement des fibres kératiniques d'une composition telle que définie précédemment.

Dans le cas où la composition comprend un ou plusieurs pigments traités en surface, le traitement des fibres kératiniques est une coloration.

Dans le cas où la composition comprend une ou plusieurs charges traitées en surface, le traitement des fibres kératiniques est un soin.

L'invention va être plus complètement illustrée à l'aide des exemples non limitatifs suivants.

### EXEMPLES

### Exemple 1 :

La composition suivante est réalisée :

| **Ingrédient** | **Quantité** |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 40 g |
| cyclopentadiméthylsiloxane commercialisé par Dow Corning sous le nom de DC245 Fluid | 40 g |
| Oxyde de fer jaune enrobé de cellulose commercialisé sous la référence C2-5 Yellow LL-100PD par la société Daito Kasei | 10 g |
| Méthylheptylcyanoacrylate de Chemence | 10 g |
| Acide acétique | 0,25 g |

0,5 g de cette composition est appliqué sur une mèche de cheveux gris à 90 % de blancs naturels propre et humide de 1 g. Après 15 minutes de pose à température ambiante, la mèche est séchée au sèche-cheveux pendant 2 minutes.

La mèche est colorée en jaune et la coloration obtenue est résistante aux shampooings. La mèche obtenue présente un toucher doux.

### Exemple 2 :

La composition suivante est réalisée :

| **Ingrédient** | **Quantité** |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 40 g |
| cyclopentadiméthylsiloxane commercialisé par Dow Corning sous le nom de DC245 Fluid | 40 g |
| Oxyde de fer jaune enrobé de C₉-C₁₅ perfluoroalkyl phosphate commercialisé sous la référence PF5 Yellow LL-100PD par la société Daito Kasei | 10 g |
| Méthylheptylcyanoacrylate de Chemence | 10 g |
| Acide acétique | 0,25 g |

0,5 g de cette composition est appliqué sur une mèche de cheveux gris à 90 % de blancs naturels propre et humide de 1 g. Après 15 minutes de pose à température ambiante, la mèche est séchée au sèche-cheveux pendant 2 minutes.

La mèche est colorée en jaune et la coloration obtenue est résistante aux shampooings. La composition tinctoriale présente une bonne stabilité dans le temps.

### Exemple 3 :

La composition suivante est réalisée :

| **Ingrédient** | **Quantité** |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 40 g |
| cyclopentadiméthylsiloxane commercialisé par Dow Corning sous le nom de DC245 Fluid | 40 g |
| Oxyde de fer jaune enrobé de polyméthylhydrogènesiloxane commercialisé sous la référence S101-2 Yellow LL-100PD par la société Daito Kasei | 10 g |
| Méthylheptylcyanoacrylate de Chemence | 10 g |
| Acide acétique | 0,25 g |

0,5 g de cette composition est appliqué sur une mèche de cheveux gris à 90 % de blancs naturels propre et humide de 1 g. Après 15 minutes de pose à température ambiante, la mèche est séchée au sèche-cheveux pendant 2 minutes.

La mèche est colorée en jaune et la coloration obtenue est résistante aux shampooings. La composition tinctoriale présente une bonne stabilité dans le temps.

### Exemple 4 :

La composition suivante est réalisée :

| **Ingrédient** | **Quantité** |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 40 g |
| cyclopentadiméthylsiloxane commercialisé par Dow Corning sous le nom de DC245 Fluid | 40 g |
| Oxyde de fer jaune enrobé de copolymère acrylate / diméthicone commercialisé sous la référence ASC-7 Yellow LL-100PD par la société Daito Kasei | 10 g |
| Méthylheptylcyanoacrylate de Chemence | 10 g |
| Acide acétique | 0,25 g |

0,5 g de cette composition est appliqué sur une mèche de cheveux gris à 90 % de blancs naturels propre et humide de 1 g. Après 15 minutes de pose à température ambiante, la mèche est séchée au sèche-cheveux pendant 2 minutes.

La mèche est colorée en jaune et la coloration obtenue est résistante aux shampooings. Les propriétés cosmétiques de la mèche obtenue sont très satisfaisantes.

### Exemple 5 :

La composition suivante est réalisée :

| **Ingrédient** | **Quantité** |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 40 g |
| cyclopentadiméthylsiloxane commercialisé par Dow Corning sous le nom de DC245 Fluid | 40 g |
| Oxyde de fer jaune enrobé d'isopropyl titanium triisostéarate commercialisé sous la référence ITT-2 Yellow LL-100PD par la société Daito Kasei | 10 g |
| Méthylheptylcyanoacrylate de Chemence | 10 g |
| Acide acétique | 0,25 g |

0,5 g de cette composition est appliqué sur une mèche de cheveux gris à 90 % de blancs naturels propre et humide de 1 g. Après 15 minutes de pose à température ambiante, la mèche est séchée au sèche-cheveux pendant 2 minutes.

La mèche est colorée en jaune et la coloration obtenue est résistante aux shampooings. Les propriétés cosmétiques de la mèche sont très satisfaisantes.

### Exemple 6 :

La composition suivante est réalisée :

| **Ingrédient** | **Quantité** |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 40 g |
| cyclopentadiméthylsiloxane commercialisé par Dow Corning sous le nom de DC245 Fluid | 40 g |
| Oxyde de fer jaune enrobé de Lauryl Lysine commercialisé sous la référence LLD-5 Yellow LL-100PD par la société Daito Kasei | 10 g |
| Méthylheptylcyanoacrylate de Chemence | 10 g |
| Acide acétique | 0,25 g |

0,5 g de cette composition est appliqué sur une mèche de cheveux gris à 90 % de blancs naturels propre et humide de 1 g. Après 15 minutes de pose à température ambiante, la mèche est séchée au sèche-cheveux pendant 2 minutes.

La mèche est colorée en jaune et la coloration obtenue est résistante aux shampooings. La mèche obtenue présente un toucher doux.

### Exemple 7 :

La composition suivante est réalisée :

| **Ingrédient** | **Quantité** |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 40 g |
| cyclopentadiméthylsiloxane commercialisé par Dow Corning sous le nom de DC245 Fluid | 40 g |
| Oxyde de fer jaune enrobé de Polyméthylhydrogène siloxane et de Phosphate de perfluoroalkyle commercialisé sous la référence FS01-52 Yellow LL-100PD par la société Daito Kasei | 10 g |
| Méthylheptylcyanoacrylate de Chemence | 10 g |
| Acide acétique | 0,25 g |

0,5 g de cette composition est appliqué sur une mèche de cheveux gris à 90 % de blancs naturels propre et humide de 1 g. Après 15 minutes de pose à température ambiante, la mèche est séchée au sèche-cheveux pendant 2 minutes.

La mèche est colorée en jaune et la coloration obtenue est résistante aux shampooings. La composition tinctoriale présente une bonne stabilité dans le temps.

### Exemple 8 :

La composition suivante est réalisée :

| **Ingrédient** | **Quantité** |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 40 g |
| cyclopentadiméthylsiloxane commercialisé par Dow Corning sous le nom de DC245 Fluid | 40 g |
| Oxyde de fer jaune enrobé d'isostéaryl sébacate commercialisé sous la référence HS-C33-8073-10 par la société Miyoshi Kasei | 10 g |
| Méthylheptylcyanoacrylate de Chemence | 10 g |
| Acide acétique | 0,25 g |

0,5 g de cette composition est appliqué sur une mèche de cheveux gris à 90 % de blancs naturels propre et humide de 1 g. Après 15 minutes de pose à température ambiante, la mèche est séchée au sèche-cheveux pendant 2 minutes.

La mèche est colorée en jaune et la coloration obtenue est résistante aux shampooings. Les propriétés cosmétiques de la mèche obtenue sont très satisfaisantes.

### Exemple 9 :

La composition suivante est réalisée :

| **Ingrédient** | **Quantité** |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 40 g |
| cyclopentadiméthylsiloxane commercialisé par Dow Corning sous le nom de DC245 Fluid | 40 g |
| Oxyde de fer jaune enrobé d'aluminium dimyristate commercialisé sous la référence MI-C33-8073-10 par la société Miyoshi Kasei | 10 g |
| Méthylheptylcyanoacrylate de Chemence | 10 g |
| Acide acétique | 0,25 g |

0,5 g de cette composition est appliqué sur une mèche de cheveux gris à 90 % de blancs naturels propre et humide de 1 g. Après 15 minutes de pose à température ambiante, la mèche est séchée au sèche-cheveux pendant 2 minutes.

La mèche est colorée en jaune et la coloration obtenue est résistante aux shampooings. Les propriétés cosmétiques de la mèche obtenue sont très satisfaisantes.

### Exemple 10 :

La composition suivante est réalisée :

| **Ingrédient** | **Quantité** |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 40 g |
| cyclopentadiméthylsiloxane commercialisé par Dow Corning sous le nom de DC245 Fluid | 40 g |
| Oxyde de fer jaune enrobé de disodium stéaroyl glutamate commercialisé sous la référence NAI-C33-8073-10 par la société Miyoshi Kasei | 10 g |
| Méthylheptylcyanoacrylate de Chemence | 10 g |
| Acide acétique | 0,25 g |

0,5 g de cette composition est appliqué sur une mèche de cheveux gris à 90 % de blancs naturels propre et humide de 1 g. Après 15 minutes de pose à température ambiante, la mèche est séchée au sèche-cheveux pendant 2 minutes.

La mèche est colorée en jaune et la coloration obtenue est résistante aux shampooings. La mèche obtenue présente un toucher naturel.

### Exemple 11 :

La composition suivante est réalisée :

| **Ingrédient** | **Quantité** |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 40 g |
| cyclopentadiméthylsiloxane commercialisé par Dow Corning sous le nom de DC245 Fluid | 40 g |
| Oxyde de fer jaune enrobé de PEG-12 diméthicone commercialisé sous la référence Yellow Iron Oxide AQ R0402 par la société LCW | 10 g |
| Méthylheptylcyanoacrylate de Chemence | 10 g |
| Acide acétique | 0,25 g |

0,5 g de cette composition est appliqué sur une mèche de cheveux gris à 90 % de blancs naturels propre et humide de 1 g. Après 15 minutes de pose à température ambiante, la mèche est séchée au sèche-cheveux pendant 2 minutes.

La mèche est colorée en jaune et la coloration obtenue est résistante aux shampooings. La mèche obtenue présente un toucher naturel.

### Exemple 12 :

La composition suivante est réalisée :

| **Ingrédient** | **Quantité** |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 40 g |
| cyclopentadiméthylsiloxane commercialisé par Dow Corning sous le nom de DC245 Fluid | 40 g |
| D&C Red 7 enrobé de méthylhydrogène polysiloxane commercialisé sous la référence SI-D&C Red 7 par la société Miyoshi Kasei | 10 g |
| Méthylheptylcyanoacrylate de Chemence | 10 g |
| Acide acétique | 0,25 g |

0,5 g de cette composition est appliqué sur une mèche de cheveux gris à 90 % de blancs naturels propre et humide de 1 g. Après 15 minutes de pose à température ambiante, la mèche est séchée au sèche-cheveux pendant 2 minutes.

La mèche est colorée en rouge et la rémanence de la coloration obtenue est bonne, notamment vis-à-vis des shampooings.

### Exemple 13 :

La composition suivante est réalisée :

| **Ingrédient** | **Quantité** |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 40 g |
| cyclopentadiméthylsiloxane commercialisé par Dow Corning sous le nom de DC245 Fluid | 40 g |
| Nacre mica - oxyde de titane - oxyde de fer enrobé d'isopropyl titanium triisostéarate commercialisé sous la référence KTZ Aruban Coral 12 par la société Taizhu | 10 g |
| Méthylheptylcyanoacrylate de Chemence | 10 g |
| Acide acétique | 0,25 g |

0,5 g de cette composition est appliqué sur une mèche de cheveux gris à 90 % de blancs naturels propre et humide de 1 g. Après 15 minutes de pose à température ambiante, la mèche est séchée au sèche-cheveux pendant 2 minutes.

La mèche est colorée en orange et la coloration obtenue est résistante aux shampooings. La mèche obtenue présente un toucher naturel.

### Exemple 14 :

La composition suivante est réalisée :

| **Ingrédient** | **Quantité** |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 40 g |
| cyclopentadiméthylsiloxane commercialisé par Dow Corning sous le nom de DC245 Fluid | 37 g |
| Mica - oxyde de fer brun commercialisé sous la référence Prestige Bronze par la société Eckart | 10 g |
| Talc enrobé de polydiméthylsiloxane commercialisé sous la référence J-68-SAT par la société US Cosmetics | 3 g |
| Méthylheptylcyanoacrylate de Chemence | 10 g |
| Acide acétique | 0,25 g |

0,5 g de cette composition est appliqué sur une mèche de cheveux gris à 90 % de blancs naturels propre et humide de 1 g. Après 15 minutes de pose à température ambiante, la mèche est séchée au sèche-cheveux pendant 2 minutes.

La mèche est colorée en bronze et la coloration obtenue est résistante aux shampooings. La mèche obtenue présente un toucher doux.

### Exemple 15 :

La composition suivante est réalisée :

| **Ingrédient** | **Quantité** |
|---|---|
| poly diméthyl siloxane alpha-omega dihydroxylé / cyclopentadiméthylsiloxane (14,7 / 85,3) commercialisé par Dow Corning sous le nom de DC 1501 Fluid | 45 g |
| cyclopentadiméthylsiloxane commercialisé par Dow Corning sous le nom de DC245 Fluid | 42 g |
| Talc enrobé de polydiméthylsiloxane commercialisé sous la référence J-68-SAT par la société US Cosmetics | 3 g |
| Méthylheptylcyanoacrylate de Chemence | 10 g |

0,5 g de cette composition est appliqué sur une mèche de cheveux gris à 90 % de blancs naturels propre et humide de 1 g. Après 15 minutes de pose à température ambiante, la mèche est séchée au sèche-cheveux pendant 2 minutes.

La mèche présente un toucher doux qui est rémanent aux shampooings.

## Revendications

1. Composition pour le traitement des matières kératiniques comprenant, dans un milieu cosmétiquement acceptable, au moins un monomère électrophile et au moins un pigment et / ou une charge préalablement traités en surface par un agent organique, ledit pigment ou ladite charge traité en surface étant différent d'un mica recouvert de titane et d'un pigment organique.

2. Composition selon la revendication 1, dans laquelle le ou les pigments sont organiques et choisis parmi les pigments de type nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

3. Composition selon la revendication 1, dans laquelle le ou les pigments sont des pigments composites composés de particules comportant un noyau inorganique recouvert au moins partiellement d'un pigment organique et au moins un liant assurant la fixation des pigments organiques sur le noyau.

4. Composition selon la revendication 1, dans laquelle le ou les pigments sont des laques constituées d'un substrat inorganique choisi parmi l'alumine, la silice, le borosilicate de calcium et de sodium, le borosilicate de calcium et d'aluminium, et l'aluminium sur lequel est adsorbé un colorant organique.

5. Composition selon la revendication 1, dans laquelle le ou les pigments sont des pigments à effets spéciaux choisis parmi les pigments nacrés, les pigments à effets interférentiels non fixés sur un substrat, les pigments fluorescents, les pigments phosphorescents, les pigments photochromiques, les pigments thermochromiques, les quantum dots.

6. Composition selon la renvendication 1, dans laquelle le ou les pigments sont des pigments nacrés choisis parmi le mica recouvert de titane et d'oxydes de fer ou d'oxyde de chrome, le mica recouvert de titane et d'un pigment organique, les pigments nacrés à base d'oxychlorure de bismuth.

7. Composition selon la revendication 1, dans laquelle le ou les pigments sont minéraux et choisis parmi les oxydes de zirconium ou de cérium, ainsi que les oxydes de fer ou de chrome, le violet de manganèse, le bleu outremer, l'hydrate de chrome et le bleu ferrique, le dioxyde de titane.

8. Composition selon l'une quelconque des revendications 1 à 7, dans laquelle la ou les charges sont choisies parmi le talc, le mica naturel ou synthétique, la silice, le kaolin, les oxychlorures de bismuth, le carbonate de calcium précipité, le carbonate et l'hydro-carbonate de magnésium, l'hydroxyapatite, les microsphères de silice creuses, les microcapsules de verre ou de céramique.

9. Composition selon l'une quelconque des revendications 1 à 7, dans laquelle la ou les charges sont choisies parmi :
- les poudres de polyamide, de poly-β-alanine et de polyéthylène, les poudres de polymères de tétrafluoroéthylène, la lauroyl-lysine, l'amidon, le nitrure de bore, les poudres de polymères d'acide acrylique ;
- les microbilles de résine de silicone ;
- les microsphères de polymères acryliques ;
- les poudres de polyuréthane ;
- les savons métalliques dérivés d'acides organiques carboxyliques ayant de 8 à 22 atomes de carbone ;
- les microcapsules de polymères ou de copolymères d'acrylate ou de méthacrylate de méthyle, ou encore de copolymères de chlorure de vinylidène et d'acrylonitrile ;
- les poudres d'organopolysiloxane réticulés élastomères.

10. Composition selon l'une quelconque des revendications précédentes, dans laquelle la ou les charges ont un diamètre apparent allant de 0,01 à 150 µm.

11. Composition selon l'une quelconque des revendications précédentes, dans laquelle l'agent organique avec lequel sont traités les pigments et les charges est choisi parmi les acides aminés ; les cires ; les acides gras, les alcools gras et leurs dérivés ; les tensio-actifs anioniques ; les lécithines ; les sels de sodium, potassium, magnésium, fer, titane, zinc ou aluminium d'acides gras ; les alcoxydes métalliques ; les polysaccharides ; le polyéthylène ; les polymères (méth)acryliques ; les polymères et copolymères contenant des motifs acrylates ; les protéines ; les alcanoamines ; les composés siliconés ; les composés organiques fluorés ; les composés fluoro-siliconés.

12. Composition selon la revendication 11, dans laquelle l'agent organique avec lequel sont traités les pigments et les charges est choisi parmi les PEG-silicones ; le chitosane ; le triéthoxycaprylylsilane ; les méthicones ; les diméthicones ; les diméthicone / triméthylsiloxysilicate ; la lauroyl lysine ; les lauroyl lysine diméthicones ; le myristate de magnésium ; le dimyristate d'aluminium ; le perfluoropolyméthylisopropyl éther ; l'isostéaryl sébacate ; le disodium stéaroyl glutamate ; les diméthicone / disodium stéaroyl glutamate ; les phosphate de perfluoroalkyle ; les associations copolymères acrylate / diméthicone et phosphate de perfluoroalkyle ; les polyméthylhydrogène siloxane / phosphate de perfluoroalkyle ; le lauryl lysine / aluminium tristéarate ; l'octyltriéthylsilane ; les associations octyltriéthylsilane et phosphate de perfluoroalkyle ; les copolymère acrylate / diméthicone ; l'isopropyl titanium triisostéarate ; la cellulose microcrystalline et la carboxyméthyl cellulose ; la cellulose ; les copolymères acrylate ; les phosphate de perfluoroalkyle / isopropyl titanium triisostéarate.

13. Composition selon l'une quelconque des revendications précédentes, dans laquelle les pigments et / ou les charges traités en surface sont recouverts par une couche organique.

14. Composition selon l'une quelconque des revendications précédentes, dans laquelle l'agent organique avec lequel sont traités les pigments et les charges peut être déposé sur lesdits pigments ou lesdites charges par évaporation de solvant, réaction chimique entre les molécules de l'agent de surface ou création d'une liaison covalente entre l'agent de surface et les pigments ou les charges.

15. Composition selon la revendication 14, dans laquelle l'agent organique avec lequel sont traités les pigments et les charges est lié auxdits pigments ou auxdites charges de manière covalente.

16. Composition selon l'une quelconque des revendications précédentes, dans laquelle l'agent avec lequel sont traités les pigments et les charges représente de 0,1 à 50 % en poids du poids total des pigments ou des charges traités en surface.

17. Composition selon l'une quelconque des revendications précédentes, dans laquelle le ou les pigments traités en surface sont présents à une concentration comprise entre 0,05 et 50 % en poids du poids total de la composition.

18. Composition selon l'une quelconque des revendications précédentes, dans laquelle la ou les charges sont présentes dans la composition dans une quantité comprise entre 0,05 et 95 % en poids du poids total de la composition.

19. Composition selon l'une quelconque des revendications précédentes, comprenant au moins une charge non traitée en surface.

20. Composition selon l'une quelconque des revendications précédentes, comprenant au moins un pigment non traité en surface.

21. Composition selon l'une quelconque des revendications précédentes, dans laquelle le ou les monomères électrophiles sont choisis parmi les composés de formule (I) : 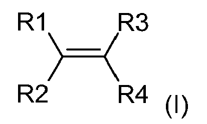 dans laquelle :
- R₁ et R₂ désignent chacun, indépendamment l'un de l'autre, un groupe peu ou non électro-attracteur ; et
- R₃ et R₄ désignent chacun, indépendamment l'un de l'autre, un groupe électro-attracteur.

22. Composition selon la revendication 21, dans laquelle R₁ et R₂ représentent, indépendamment l'un de l'autre, un atome d'hydrogène ; un groupe hydrocarboné saturé ou non, linéaire, ramifié ou cyclique, comportant de 1 à 20 atomes de carbone et contenant éventuellement un ou plusieurs atomes d'azote, d'oxygène, de soufre, et éventuellement substitué par un ou plusieurs groupements choisis parmi -OR, -COOR,-COR, -SH, -SR, -OH et les atomes d'halogène ; un résidu polyorganosiloxane modifié ou non ; un groupement polyoxyalkylène ; R désignant un groupe hydrocarboné saturé ou non, linéaire, ramifié ou cyclique, comportant de 1 à 20 atomes de carbone et contenant éventuellement un ou plusieurs atomes d'azote, d'oxygène, de soufre, et éventuellement substitué par un ou plusieurs groupements choisis parmi-OR', -COOR', -COR', -SH, -SR', -OH et les atomes d'halogène, ou un résidu de polymère, R' étant un radical alkyle en C₁-C₁₀.

23. Composition selon la revendication 21 ou 22, dans laquelle R₃ et R₄ sont choisis, indépendamment l'un de l'autre, parmi les groupements -N(R)₃⁺, -S(R)₂⁺, -SH₂⁺, -NH₃⁺, -NO₂, -SO₂R, -C≡N, -COOH, -COOR, -COSR, -CONH₂, -CONHR, -F, -Cl, -Br, -I, -OR, -COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle et aryloxy ; R désignant un groupe hydrocarboné saturé ou non, linéaire, ramifié ou cyclique, comportant de 1 à 20 atomes de carbone et contenant éventuellement un ou plusieurs atomes d'azote, d'oxygène, de soufre, et éventuellement substitué par un ou plusieurs groupements choisis parmi -OR', -COOR', -COR', -SH, -SR', -OH et les atomes d'halogène, ou un résidu de polymère, R' étant un radical alkyle en C₁-C₁₀.

24. Composition selon l'une quelconque des revendications précédentes, dans laquelle le ou les monomères électrophiles sont choisis parmi les monomères de la famille des cyanoacrylates de formule (II) : 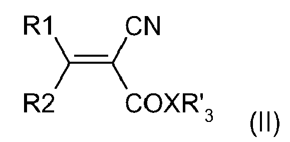 dans laquelle :
- X peut désigner NH, S, O ;
- R'₃ peut désigner un atome d'hydrogène ou un groupe R ;
- R, R₁ et R₂ sont tels que définis dans la revendication 22.

25. Composition selon l'une quelconque des revendications 21 à 24, dans laquelle R₁ et R₂ représentent un atome d'hydrogène.

26. Composition selon la revendication 24 ou 25, dans laquelle X désigne O.

27. Composition selon l'une quelconque des revendications 24 à 26, dans laquelle R'₃ représente un radical alkyle en C₆-C₁₀.

28. Composition selon l'une quelconque des revendications précédentes, dans laquelle le ou les monomères électrophiles sont choisis parmi les monomères de la famille des 2-cyanoacrylates d'alkyle ou d'alcoxyalkyle de formule (VI) : 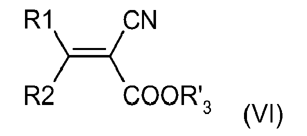 dans laquelle :
- R'₃ représente un radical alkyle en C₁-C₁₀, alcényle en C₂-C₁₀ ou alcoxy(C₁-C₄)alkyle(C₁-C₁₀) ;
- R₁ et R₂ sont tels que définis à la revendication 22.

29. Composition selon la revendication 28, dans laquelle R'₃ représente un radical alkyle en C₆-C₁₀.

30. Composition selon la revendication 28 ou 29, dans laquelle R₁ et R₂ représentent un atome d'hydrogène.

31. Composition selon l'une quelconque des revendications précédentes, dans laquelle le ou les monomères électrophiles sont choisis parmi les composés de formule (VII) et leurs mélanges : 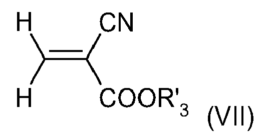 dans laquelle R'₃ est choisi parmi les radicaux suivants :
-(CH₂)₇-CH₃ ;
-CH(CH₃)-(CH₂)₅-CH₃ ;
-CH₂-CH(C₂H₅)-(CH₂)₃-CH₃ ;
-(CH₂)₅-CH(CH₃)-CH₃ ;
-(CH₂)₄-CH(C₂H₅)-CH₃.

32. Composition selon l'une quelconque des revendications précédentes, dans laquelle le ou les monomères électrophiles sont présents en quantité comprise entre 0,1 % et 80 % en poids environ du poids total de la composition.

33. Composition selon l'une quelconque des revendications précédentes, dans laquelle le milieu cosmétiquement acceptable est anhydre.

34. Composition selon la revendication 33, dans laquelle le milieu cosmétiquement acceptable est choisi parmi :
• les alcools aromatiques ;
• les alcools gras ;
• les polyols modifiés ou non modifiés ;
• les silicones volatiles ou non volatiles ;
• les huiles minérales, organiques ou végétales ;
• les cires oxyéthylénées ou non oxyéthylénées, les paraffines, les alcanes ;
• les acides gras, les amides grasses, les esters gras ; et leurs mélanges.

35. Composition selon l'une quelconque des revendications précédentes, comprenant de plus au moins un agent nucléophile.

36. Composition selon la revendication 35, dans laquelle l'agent nucléophile est l'eau.

37. Procédé pour le traitement des fibres kératiniques, comprenant l'application sur les fibres kératiniques d'une composition telle que définie à l'une quelconque des revendications 1 à 34 en présence d'au moins un agent nucléophile.

38. Procédé selon la revendication 37, dans lequel l'application de la composition pour la coloration des fibres kératiniques est réalisée en au moins deux étapes, dans lequel une des étapes consiste à appliquer sur les fibres kératiniques une composition comprenant le ou les pigments et / ou la ou les charges traités en surface et l'autre étape consiste à appliquer sur les fibres kératiniques une composition comprenant le ou les monomères électrophiles, l'ordre des étapes étant indifférent.

39. Procédé selon la revendication 37 ou 38, dans lequel l'agent nucléophile est appliqué séparément.

40. Procédé selon la revendication 37 ou 38, dans lequel l'agent nucléophile est présent dans la composition comprenant le ou les pigments traités en surface.

41. Procédé selon l'une quelconque des revendications 37 à 40, comprenant une étape qui consiste à appliquer sur les fibres kératiniques au moins un agent réducteur de la kératine, avant l'application de la composition pour la coloration des matières.

42. Kit pour le traitement des fibres kératiniques, **caractérisé par le fait qu'**il contient une composition comprenant au moins un monomère électrophile tel que défini à l'une quelconque des revendications 1 et 21 à 31 et éventuellement au moins un inhibiteur de polymérisation et une composition comprenant au moins un pigment et / ou une charge préalablement traités en surface tels que définis à l'une quelconque des revendications 1 à 16.

43. Kit selon la revendication 42, dans lequel la composition comprenant le ou les pigments et / ou la ou les charges traités en surface comprend de plus au moins un agent nucléophile tel que défini à la revendication 35 ou 36.

44. Kit selon la revendication 42, contenant de plus une composition comprenant au moins un agent nucléophile tel que défini à la revendication 35 ou 36.

45. Kit selon l'une quelconque des revendications 42 à 44, dans lequel la composition comprenant le ou les pigments et / ou la ou les charges traités en surface et la composition comprenant le ou les monomères électrophiles et éventuellement le ou les inhibiteurs de polymérisation sont présentes dans une seule composition anhydre.

46. Kit selon l'une quelconque des revendications 42 à 44, dans lequel la composition comprenant le ou les pigments et / ou la ou les charges traités en surface est une composition aqueuse et la composition comprenant le ou les monomères électrophiles et éventuellement le ou les inhibiteurs de polymérisation est une composition anhydre.

47. Utilisation pour le traitement des fibres kératiniques d'une composition telle que définie à l'une quelconque des revendications 1 à 36.
